# EUROPÄISCHE PATENTSCHRIFT

(11) **EP 0 446 826 B1**
(45) Veröffentlichungstag und Bekanntmachung des Hinweises auf die Patenterteilung: **15.11.1995**
(21) Anmeldenummer: 91103667.1
(22) Anmeldetag: 11.03.1991
(51) Int. Cl.: C12P 13/00, C12M 1/40

(54) **Verfahren zur enzymatischen Herstellung optisch aktiver Cyanhydrine**
Process for the production of optical active cyanhydrin
Procédé pour la préparation enzymatique de cyannydrine optiquement active

(30) Priorität: 16.03.1990 DE 4008412; 10.09.1990 DE 4028689; 16.03.1990 DE 4008411
(43) Veröffentlichungstag der Anmeldung: 18.09.1991
(73) Patentinhaber: FORSCHUNGSZENTRUM JÜLICH GMBH, 52425 Jülich (DE)
(72) Erfinder: Miethe, Peter, Dr., O-4020 Halle (DE); Kula, Maria Regina, Prof., W-5162 Niederzier-Hambach (DE); Stürtz, Ingeborg Maria, W-5180 Eschweiler (DE); Wandrey, Christian, Prof. Dr., W-5170 Jülich (DE); Kragl, Udo, W-5170 Jülich (DE)

(56) Entgegenhaltungen:
- EP-A- 0 276 375
- EP-A- 0 326 063
- EP-A- 0 340 744
- EP-A- 0 347 873
- EP-A- 0 350 908
- WO-A-88/07075
- ANGEWANDTE CHEMIE, Internatl. Ed. in Englisch, Band 24, Nr. 6, 1985; Seiten 439-450

## Beschreibung

Die Erfindung bezieht sich auf ein Verfahren zur Herstellung von optisch aktiven Cyanhydrinen durch enzymatische Umsetzung von Oxonverbindungen mit Blausäure in Gegenwart von (R)- bzw. (S)-Oxynitrilase (4.1.2.10) bzw. (4.1.2.11) unter derart sauren Bedingungen, daß die chemische Konkurrenzreaktion und die Racemisierung vernachlässigbar sind.

Optisch aktive Cyanhydrine sind für die Gewinnung von optisch aktiven α-Aminoalkoholen, α₋Hydroxycarbonsäuren, Heterocyclen und Pyrethroid-Insektiziden von erheblichem Interesse. Dabei ist die Verfügbarkeit von leicht derivatisierbaren chiralen Synthesebausteinen, die kostengünstig in ausreichenden Mengen mit möglichst hohem Enantiomerenexzeß (ee) hergestellt werden können, von zentraler Bedeutung.

In Anbetracht der optischen Selektivität enzymatischer Reaktionen wurde die enzymkatalysierte Herstellung optisch aktiver Cyanhydrine schon seit langem untersucht: So wird in der DE-PS 13 00 111 die Herstellung von optisch aktiven Cyanhydrinen mit an Ionenaustauschern gebundener (R)-Oxynitrilase bei pH 5,4 beschrieben. Die dabei erzielten ee-Werte lagen allerdings durchweg unter 90 %.

Von Effenberger et al (Angew. Chem. 99 (1987) 491) wird daher die enzymatische Umsetzung von Oxoverbindungen mit Blausäure in organischen mit Wasser nicht mischbaren Lösungsmitteln empfohlen, um die chemische Reaktion zu unterdrücken. Dabei wurde bevorzugt mit trägerfixiertem Enzym in Ethylacetat bei pH-Werten von 5,4 gearbeitet. Dabei wurden ee-Werte bis zu 99 % erzielt.

Ein anderer Weg, die chemische Konkurrenzreaktion und Racemisierung zu unterdrücken, wird in der EP A2 0 326 063, angegeben, nach der optisch aktive Cyanhydrine durch Umsetzung von Oxoverbindungen mit Slausäure in Gegenwart von Oxynitrilase erhalten werden sollen, indem unter derart sauren Bedingungen, insbesondere bei pH-Werten ≦ 4,5 und bei solchen Temperaturen gearbeitet wird, daß die chemische Konkurrenzreaktion und die Racemisierung gegenüber der enzymatischen Synthese vernachlässigbar sind. Auf die unter diesen Bedingungen erhöhten Aktivitätsverluste des Biokatalysators wird dabei hingewiesen und die Beispiele lassen eine Favorisierung niedriger Temperaturen im Bereich von 5 bis 8°C erkennen.

Es wurde nun überraschenderweise festgestellt, daß die Bevorzugung der enzymatischen Synthese vor der chemischen Konkurrenzreaktion und Racemisierungen im sauren Bereich bei zum Neutralpunkt hin verschobenen pH-Werten (und auch bei Zimmertemperatur) erreicht werden kann, wenn der Biokatalysator in einem lyotropen Flüssigkristall solubilisiert vorliegt und die Substratzufuhr über eine organische Lösungsmittelphase erfolgt.

Das erfindungsgemäße Verfahren der eingangs genannten Art ist daher dadurch gekennzeichnet, daß man die Umsetzung in einem organischen Lösungsmittel in Gegenwart von in einem lyotropen Flüssigkristall solubilisierter Oxynitrilase durchführt, wobei für die Flüssigkristallbildung solche Tenside ausgeschlossen werden, deren Hydrolyse zu einer Erhöhung des pH-Wertes führt.

Auf diese Weise können optisch aktive (R)-Cyanhydrine und (S)-Cyanhydrine durch Umsetzung von aliphatischen, aromatischen oder heteroaromatischen Aldehyden oder Ketonen mit Blausäure in Gegenwart der im lyotropen Flüssigkristall "immobilisierten" (R)- bzw. (S)-Oxynitrilase erhalten werden. Dabei wird für die Erzeugung der flüssigkristallinen Phase, insbesondere Pufferlösung verwendet, deren pH-Wert zwischen 3 und 6 liegt.

Als Tenside werden kationische oder nichtionische Amphiphile bevorzugt, wie Alkyltrimethylammoniumhalogenide, Alkylpyridiniumhalogenide, Polyoxyethylenether, Polyoxyethylenester, Polyoxyethylensorbitanester, Alkylphenolpolyethylenglykolether, entsprechende Polyoxypropylenderivate oder Copolyoxyethylenpolyoxypropylenderivate oder entsprechende Tensidgemische und als organische Lösungsmittel sind insbesondere Methylenchlorid, Chloroform, Tetrachlorkohlenstoff, Dibutylether, Diisopropylether oder Toluol zweckmäßig. In der Praxis haben sich Dibutylether und Brij® 35 (Polyoxyethylenmonolaurylether) der Fa. Serva gut bewährt.

Zur Herstellung des flüssigkristallinen biokatalysatorhaltigen Systems geht man zweckmäßigerweise von einer 1 bis 30 Gew.%-igen, vorzugsweise 5 bis 10 Gew.%-igen Stammlösung bzw. Suspension eines Tensids in einem organischen Lösungsmittel aus. Zu dieser Stammlösung werden nachfolgend 1 bis 30 Gew.%, insbesondere 5 bis 10 Gew.% wäßrige biokatalysatorhaltige Pufferlösung hinzugegeben. Nach kurzem Schütteln entsteht daraus das biphasige System Flüssigkristall/organisches Lösungsmittel.

Die exakte Zusammensetzung eines solchen Systems muß in entsprechenden Vorversuchen ermittelt werden, sofern sie nicht der kolloidchemischen Literatur (ternäre Phasendiagramme Tensid/organisches Lösungsmitttel/Wasser) entnommen werden kann.

Die Erfindung eignet sich insbesondere für die Umsetzung wasserunlöslicher Oxoverbindungen. Im Falle, daß die Oxoverbindung nicht zu polar ist, kann diese selbst das organische Lösungsmittel bilden.

Die biokatalysatorhaltigen lyotropen Flüssigkristalle werden vorzugsweise in Mischung mit porösen Trägermaterialien, wie z. B. porösen Glassinterkörpern verwendet, wobei der Einsatz in Form einer damit gepackten Durchlaufsäule besonders zweckmäßig ist.

Verfahrenstechnisch besonders günstig ist auch ein Durchlaufreaktor, der dünne Schichten des biokatalysatorhaltigen Flüssigkristalls angrenzend an schmale Strömungskanäle für substrathaltiges Lösungsmittel aufweist.

Zwar wird die enzymatische Umsetzung von organischen Verbindungen in Gegenwart von lyotropen Flüssigkristallen (vorzugsweise mit inverser Phasenstruktur), die das Enzym enthalten, bereits in der EP A2 0 340 744 beschrieben, jedoch war danach nicht erkennbar, daß bei der Herstellung optisch aktiver Cyanhydrine auf enzymatischem Wege durch den Einsatz von lyotropen Flüssigkristallen eine pH-Verschiebung des optimalen Umsetzungsbereiches möglich sein würde, die eine erfolgreiche Umsetzung unter günstigen Bedingungen (insbesondere ohne die Notwendigkeit, Kühltemperaturen einzusetzen) und mit relativ geringen Enzymverlusten möglich machen würde. Selbstverständlich kann auch die erfindungsgemäße Umsetzung innerhalb eines relativ breiten Temperaturbereichs von etwa -10°C bis 70°C stattfinden.

Überraschenderweise können in Falle der Oxynitrilase neben den bisher vorzugsweise verwendeten Flüssigkristallen mit inverser Phasenstruktur gleichwertig auch solche mit normaler Phasenstruktur eingesetzt werden. Diese Tatsache vereinfacht die Auswahl und erweitert die Möglichkeiten ein flüssigkristallines Reaktionssystem herzustellen erheblich.

Nachfolgend wird die Erfindung anhand von Beispielen beschrieben:

### Beispiel 1

Es wurde eine Stammlösung von 10 Gew.% Decaethylenglycoldodecylether in Dibutylether hergestellt. Zu 5 ml dieser Lösung wurden 280 µl einer wäßrigen R-Oxynitrilaselösung (Proteinkonzentration 10 mg/ml, 50 mM Citratpuffer pH = 3,75) gegeben. Nach kurzem Schütteln entsteht ein das Enzym enthaltender Flüssigkristall im Gleichgewicht mit reinem Dibutylether. Das Volumenverhältnis der beiden Phasen beträgt etwa 1 : 1. In dieses System wurden anschließend 100 mg Benzaldehyd und 100 µl Blausäure gegeben, und die einsetzende Cyanhydrinsynthese wurde polarimetrisch im Dibutylether verfolgt. Nach 50 min stellte sich ein konstanter Drehwert ein, und die Reaktion war beendet. Dann wurde die organische Phase vom Flüssigkristall abgetrennt, und das Lösungsmittel wurde im Rotationsverdampfer abgezogen.
Chem. Ausbeute 115 mg (90 % der Th.)
Optische Reinheit 99 % ee
Die Bestimmung der optischen Reinheit erfolgte als N,O-Bis-(Pentafluorpropionyl)-2-amino-1-phenylethanol-Derivat des R-Mandelsäurenitrils kapillargaschromatographisch nach H. Frank et al. (J. Chromatogr. 146, (1987), 197-206) auf einer chiralen Trennphase (FS-Chirasil-Val, 25 m x 0,32 mm).

Die Derivatisierung wurde wie folgt vorgenommen: 1 - 2 mg des Mandelsäurenitrils wurden mit 250 µl einer 1 M Diboranlösung (in Tetrahydrofuran) in Dibutylether bei Zimmertemperatur in 30 min reduziert. Nach Hydrolyse des überschüssigen Diborans mit einigen Tropfen Ethanol und Abziehen des Lösungsmittels wurde der erhaltene Aminoalkohol direkt mit 20 µl Pentafluorpropionsäureanhydrid in Methylenchlorid bei Zimmertemperatur in 15 min acyliert. Schließlich wurde überschüssiges Anhydrid am Rotationsdampfer abgezogen, der Rückstand mit Methylenchlorid wieder aufgenommen und gaschromatographisch analysiert.

### Beispiel 2

Entsprechend Beispiel 1 wurden 100 mg 3-Phenoxybenzaldehyd umgesetzt. Nach ca. 90 min war die Reaktion beendet. Die Aufarbeitung des Cyanhydrins und die Bestimmung der optischen Reinheit erfolgte, wie in Beispiel 1 beschrieben.
Chem. Ausbeute 90 mg (87 % d. Th.)
Optische Reinheit 99 % ee

### Beispiel 3

Entsprechend Beispiel 1 wurden 100 mg 3-Fluorbenzaldehyd umgesetzt. Die Reaktion war nach 90 min beendet. Die Aufarbeitung erfolgte analog zu Beispiel 1.
Chem. Ausbeute 102 mg (83 % d. Th.)
Optische Reinheit 76 % ee

### Beispiel 4

Entsprechend Beispiel 1 wurden 100 mg 2-Methylcyclohexanon umgesetzt. Die Reaktion war nach ca. 120 min beendet. Aufarbeitung und Analytik erfolgten analog zu Beispiel 1.
Chem. Ausbeute 111 mg (82 % d. Th.)
Optische Reinheit 92 % de

### Beispiel 5

Kontinuierliche Produktion von (R)-Mandelsäurenitril im Festbettreaktor.

Es wurde eine Lösung von 10 Gew.% Brij 35 (SIGMA) in Diisoprophylether hergestellt. Zu 20 ml dieser Lösung wurden 700 µl einer wäßrigen (R)-Oxynitrilaselösung gegeben (Proteinkonzentration 10 mg/ml, Citratpuffer pH = 4). Nach kurzem Schütteln entstehen daraus etwa 6 g lyotroper Flüssigkristall im Gleichgewicht mit Diisopropylether. 3 g von diesem flüssigkristallinen Biokatalysator wurden in den Innenraum von 10 Siran-Raschigringen (Innendurchmesser 5 mm, Länge 8 mm) gefüllt. Diese Siranringe wurden in eine 25 ml Chromatographiesäule gegeben, der Säulenausfluß wurde an eine Pumpe mit anschließender Blasenfalle und polarimetrischer Durchflußzelle angeschlossen, und das System wurde mit 30 ml Diisopropylether gefüllt. Nachdem der Säulenzufluß mit einer eisgekühlten Vorlage, die eine Diisopropyletherlösung mit 25 µl/ml Benzaldehyd und 25 µl/ml HCN enthielt, gekoppelt worden war, wurde die Reaktion in der Durchflußapparatur durch Einschalten der Pumpe gestartet. Nach ca. 140 min war der steady state erreicht. Die Apparatur wurde 2 Tage kontinuierlich betrieben, wobei kein Absinken des Drehwertes zu verzeichnen war.

### Betriebsbedingungen:

- Benzaldehyd:: 250 mM
- HCN:: 1 M
- Verweilzeit:: 80 min
- mittlerer Umsatz:: 85 %
- Raumzeitausbeute:: 550 g/(l d)
- optische Reinheit:: 99 % ee

### Beispiel 6

Kontinuierliche Produktion von (R)-Mandelsäurenitril in Festbettreaktor mit unterschiedlichen Verweilzeiten.

### Versuchsbeschreibung:

- Ansatz:: 2,5 g Brij 35
10 ml Dibutylether
3,5 ml R-Oxynitrilase in 50 mM Citratpuffer pH 3,75 (110 U/ml; 385 U im Ansatz)
70 g Glasperlen
- Substratkonzentrationen:: 90 mM Benzaldehyd 200 mM Blausäure
- Temperatur:: 25 °C
- Polarimeter:: λ = 436 nm
- Reaktorvolumen::
- gesamtes Volumen:: 50 ml
- durchströmbares Volumen:: 7,0 ml

Es wurde eine Lösung von 25 Gew.% Brij 35 (SIGMA) in Dibutylether herstellt. Zu 10 ml dieser Lösung wurden 3,5 ml einer wässrigen (R)-Oxynitrilaselösung gegeben (Proteinkonzentration 10 mg/ml, Citratpuffer PH 3,75). Nach kurzem Schütteln entstehen daraus etwa 6 g lyotroper Flüssigkristall im Gleichgewicht mit Dibutylether. Der flüssigkristalline Biokatalysator wurde mit 70 g Glasperlen vermischt und in eine 50 ml Chromatographiesäule gegeben. Der Säulenausfluß wurde an eine Pumpe mit anschließender Blasenfalle und polarimetrischer Durchflußzelle angeschlossen, und das System wurde mit 50 ml Dibutylether gefüllt. Nachdem der Säulenzufluß mit einer eisgekühlen Vorlage, die eine Dibutyletherlösung mit 90 mM Benzaldehyd,und 200 mM Blausäure enthielt, gekoppelt worden war, wurde die Reaktion in der Durchflußapparatur durch Einschalten der Pumpe gestartet. Es wurden verschiedene Verweilzeiten eingestellt und jeweils gewartet bis der steady state erreicht war. Die Apparatur wurde 3 Tage kontinuierlich betrieben, wobei kaum ein Absinken des Drehwertes zu verzeichnen war.

Für die Berechnung der Verweilzeit und der Raum-Zeit-Ausbeute wurde das durchströmbare Volumen benutzt.

Es wurde der Umsatz und der Drehwert bei 10 verschiedenen Verweilzeiten bestimmt.

| Flow ml/min | α grd | VWZ min | c[BA] mM | c[MSN] mM | Umsatz % | RZ-Ausbeute g/l·d | ee % |
|---|---|---|---|---|---|---|---|
| 0,1 | 0,262 | 70 | 2 | 83 | 98 | 226 | 96 |
| 0,2 | 0,262 | 35 | 2 | 83 | 98 | 452 | 96 |
| 0,4 | 0,263 | 17,5 | 2 | 83 | 98 | 905 | 96 |
| 0,8 | 0,232 | 9 | 10 | 75 | 88 | 1600 | 95 |
| 1,6 | 0,183 | 4,4 | 32 | 61 | 72 | 2650 | 93,5 |
| 2,0 | 0,153 | 3,5 | 42 | 48 | 56 | 2633 | 96,5 |
| 2,4 | 0,112 | 3 | 61 | 32 | 38 | 2064 | 94 |
| 3,2 | 0,080 | 2,2 | 69 | 25 | 30 | 2160 | n.b. |
| 4,0 | 0,068 | 1,75 | 67 | 21 | 26 | 2312 | n.b. |
| 4,8 | 0,054 | 1,6 | 73 | 17 | 20 | 2070 | n.b. |
| BA - Benzaldehyd MSN - Mandelsäurenitril | | | | | | | |

Wie die vorstehende Tabelle erkennen läßt, kann die Mandelsäurenitrilbildung optimiert werden: Bei der durchgeführten Versuchsreibe wurden bei Verweilzeiten zwischen 3 und 4 Min. die höchsten Raum-Zeit-Ausbeuten bei praktisch unverminderter Enantiomeren-Reinheit erhalten.

### Beispiel 7

### Enzymstabilität

Es wurden 40 (R)-Oxynitrilase enthaltende flüssigkristalline Systeme entsprechend Beispiel 1 hergestellt und in 5 ml Reagenzgläsern mit Schliff gefüllt. In dieser Form wurde die Oxynitrilase bei Zimmertemperatur gelagert, und die Aktivität des Enzyms wurde jeweils nach einem Tag gemessen. Dazu wurden 100 µl Benzaldehyd und 100 µl HCN in jeweils eines dieser Gläser gegeben, und der Drehwert wurde diskontinuierlich nach 10, 20 und 40 min gemessen. Der Anstieg dieser Drehwert-Zeit-kurve wurde als Maß für die Enzymaktivität verwendet. Innerhalb der ersten 4 Tage war ein Abfall von etwa 3 % pro Tag zu verzeichnen. Danach war über einen Zeitraum von 40 Tagen kein weiterer Aktivitätsverlust zu verzeichnen, und die Enzymaktivität lag bei etwa 80 % der Ausgangsaktivität. Im Citratpuffer bei pH = 3,75 und 20°C verliert die Oxynitrilase eine Aktivität etwa 8 % pro Tag.

### Beispiel 8

### Synthese bei pH 4 bis 6,5

Es wurde verfahren wie in Beispiel 5 dargestellt, wobei verschiedene wäßrige enzymhaltige Lösungen bei pH = 4,0; 4,5; 5,0; 5,5; 6,0; 6,5 eingesetzt wurden. Nach 24 h kontinuierlichem Betrieb wurde jeweils die optische Reinheit des gebildeten Mandelsäurenitrils bestimmt. In nachfolgender Tabelle sind die erhaltenen Werte zusammengefaßt:

| pH | 3,0 | 4 | 5 | 6 | 7 |
|---|---|---|---|---|---|
| % ee | 96,4 | 97,2 | 92,7 | 89 | 88,1 |

### Beispiel 9

Die Umsetzung wurde in einem 100 ml Zweihalskolben mit KPG Rührer ausgeführt. Um das Reaktionssystem in eine rührfähige Form zu überführen, bei dem insbesondere das Ankleben des Flüssigkristalls an den Rührerblättern vermieden werden kann, wurden folgende Varianten entwickelt:
a) 2g Polyethylenglycol-20-dodecylether (Brij 35) werden bei 50°C mit 40 ml Dibutylether gemischt. Es entsteht eine Stammlösung von Tensid in Dibutylether. Nach Abkühlung auf 35°C werden 2,8 ml Enzymlösung (Proteinkonzentration 18 mg/ml) zugegeben. Durch kräftiges Schütteln erzeugt man etwa 2 ml Flüssigkristall. Zu diesem gibt man 50 ml nichtporöse Glaskugeln (d= 2-4 mm).
b) Es wird verfahren wie unter a) beschrieben. An Stelle der nichtporösen Glaskugeln werden jedoch 40 ml poröse Siranglaskugeln (Schott d=0,2-0,6 mm, Poren 120 µm) zugegeben. Das System wird kurzzeitig evakuiert, um Luftblasen zu entfernen. Die Aufnahme der Flüssigkristallmasse in die Hohlräume des porösen Glases wird ggf. durch anschließende Druckanwendung (z.B. 2 bar) gefördert.
c) Es wird verfahren wie unter a) beschrieben. An Stelle von nichtporösen Glaskugeln werden jedoch 50 ml Seesand oder 50 ml Cellulosepulver (Avicell, FMC) oder 50 ml getrocknete Perlzellulose (Leipziger Arzneimittelwerk) zugegeben.
d) Bei Einsatz von porösen Materialien d.h. Cellulosepulver, Perlzellulose, Siranglas kann auch wie folgt verfahren werden. Zu 10 g des Trägermaterials gibt man 3 ml der Enzymlösung. Dieses feuchte Trägermaterial gibt man anschließend in 40 ml der unter a) beschriebenen Stammlösung von Brij 35 in Dibutylether. Bei diesem Verfahren kann der Wassergehalt im Bereich von 2,6 bis 8 ml pro Ansatz variiert werden.
e) 40 g Eupergit® C 250 L (Röhm Pharma) werden mit 200 ml R-Oxynitrilaselösung (1,9 g/l; pH=7,5) versetzt und 72 h bei Zimmertemperatur stehen lassen. Anschließend wird mit dest. Wasser gewaschen und das Immobilisat 24 h bei Zimmertemperatur im Vakuum getrocknet. 40g Immobilisat werden dann mit 6ml Citratpuffer von pH 3,75 benetzt und anschließend in 50 ml der unter a) beschriebenen Stammlösung dispergiert. Der Wassergehalt des Systems kann in den Grenzen von 2 bis 10 ml pro Ansatz variiert werden.

In allen Fällen wurde bei einer Rührergeschwindigkeit von 150 rpm bei Zimmertemperatur gearbeitet. Es wurden jeweils 20 ml wassergesättiger Dibutylether, 2 ml Benzaldehyd und 2 ml Blausäure zugesetzt. Nach einer Reaktionszeit von 12 h wurde mit Hilfe der HPLC (Säule RP-18, Laufmittel Acetonitril/Citratpuffer pH=4,2 = 30/70) der Umsatz und wie im Beispiel 1 beschrieben der ee-Wert bestimmt. Dabei wurde gefunden:
Variante a) Umsatz 90 %; ee=97,2 %
Variante b) Umsatz 86 %; ee=96,0 %
Variante c) Seesand: Umsatz 87 %; ee=92,7 %
Variante c) Avicell: Umsatz 75 %; ee=91,0 %
Variante c) Perlzellulose: Umsatz 78 %; ee=88 %
Variante d) Umsatz 66 %; ee=81 %
Variante e) Umsatz 56 %; ee=78,8 %
Als Reaktor für solche Umsetzungen mit Hilfe von Biokatalysatoren, die in Form lyotroper Flüssigkristalle eingesetzt werden, ist z.B. ein Durchflußreaktor zweckmäßig, bei dem flüssigkristallhaltige Schichten mit Strömungskanälen für substrathaltige Flüssigkeit abwechseln, die zumindest teilweise durch flüssigkeitsdurchlässige Schichten, aus porösem Material gebildet werden, das die flüssigkristallhaltigen Schichten begrenzt, deren Abmessungen quer zu den Strömungskanälen eine im wesentlichen vollständige Durchdringung der gesamten flüssigkristallhaltigen Schicht durch reaktives Substrat gestatten.

Die Strömungkanäle werden insbesondere durch Schichten von flüssigkeitsdurchlässigem Sintermaterial gebildet und füllen vorzugsweise < 50 %, insbesondere < 30 % des Reaktorvolumens aus.

Die flüssikristallhaltige Schicht wird insbesondere durch eine Mischung von Biokatalysator enthaltenden Flüssigkristallen mit porösem Trägermaterial, insbesondere porösem Sinterglas gebildet und hat vorzugsweise ein Schichtdicke von < 1 cm. Diejenige der Strömungskanäle ist vorzugsweise < 0,5 cm.

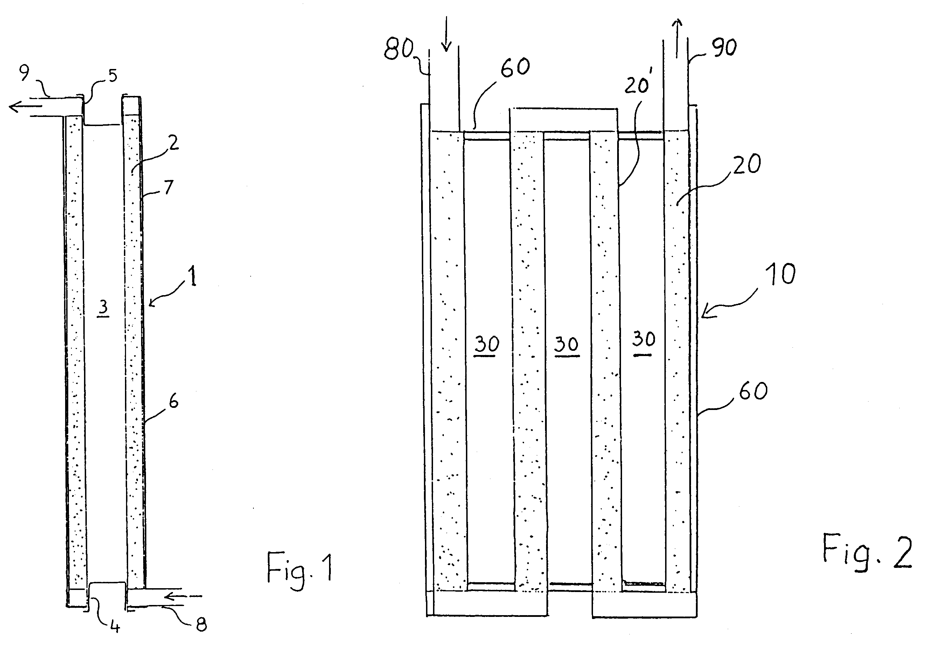

Geeignete Anordnungen werden schematisch durch Figur 1 und 2 wiedergegeben.

Figur 1 zeigt einen Reaktor 1, dessen poröses Rohr, 2 eine Biokatalysator aufweisende Flüssigkristallfüllung 3 enthält und stirnseitig durch Kappen, Stopfen oder dergleichen 4, 5 abgeschlossen ist. Das Rohr 2 paßt gleitend in das Hüllrohr 6, das den Reaktormantel bildet und einen Strömungskanal für Flüssigkeitsströmung bildet, der durch den Spalt 7 zusammen mit dem Porenvolumen des Rohres 2 gebildet wird. Die Flüssigkeit fließt über einen Zulaufverteiler 8 zu und gelangt über 9 aus dem Reaktor. Endabschluß und Flüssigkeitsverteiler sind nicht näher ausgeführt.

Figur 2 zeigt im Schema den Aufbau eines Plattenmodulreaktors 10, der durch eine Serie von Platten 20 gebildet wird, die gleichsinnig oder alternierend (wie skizziert) durchströmt werden können und zwischen denen Flüssigkristall-Schichten 30 vorgesehen sind.

Am einfachsten wird ein solcher aus einer Vielzahl von Platten bestehender Reaktor durch einen Stapel von voneinander unabhängigen Platten 20 gebildet, deren Längskanten abgedichtet mit der Reaktorwand 60 abgeschlossen sind. Auf die großen Flächen 20' der Platten 20 sind ein- oder beidseitig Flüssigkristall-Schichten aufgetragen, deren Mächtigkeit den Zwischenraum zwischen den Platten bestimmt, wobei ggf. Abstandshalter für die Einhaltung des einmal gewählten Abstandes sorgen.

## Patentansprüche

1. Verfahren zur Herstellung von optisch aktiven Cyanhydrinen durch enzymatische Umsetzung von Oxoverbindungen mit Blausäure in Gegenwart von (R)- bzw. (S)-Oxynitrilase (4.1.2.10) bzw. (4.1.2.11) unter derart sauren Bedingungen, daß die chemische Konkurrenzreaktion und die Racemisierung vernachlässigbar sind,
**dadurch gekennzeichnet,**
daß man die Umsetzung in einem organischen Lösungsmittel in Gegenwart von in einem lyotropen Flüssigkristall solubilisierter Oxynitrilase durchführt, wobei für die Flüssigkristallbildung solche Tenside ausgeschlossen werden, deren Hydrolyse zu einer Erhöhung des pH-Wertes führt.

2. Verfahren nach Anspruch 1,
**dadurch gekennzeichnet,**
daß man die Umsetzung in einem solchen ternären System von Tensid / organischem Lösungsmittel / wäßrigem Puffer durchführt, das unter Verwendung von Pufferlösungen mit pH-Werten zwischen 3 und 6 hergestellt worden ist.

3. Verfahren nach Anspruch 1 oder 2,
**dadurch gekennzeichnet,**
daß man die Umsetzung in einem Durchlaufreaktor mit an porösem Träger, insbesondere Glasträger fixiertem Flüssigkristall durchführt.

4. Verfahren nach einem der vorangehenden Ansprüche,
**dadurch gekennzeichnet,**
daß man die Umsetzung in einem Durchflußreaktor durchführt, der den Flüssigkristall in dünner Schichtung angrenzend an schmale, eine flüssigkeitsdurchlässige Begrenzung aufweisende Strömungskanäle enthält, durch die substrathaltiges Lösungsmittel bzw. Lösungsmittelgemisch tangential zur Flüssigkristallschichtung strömend geschickt wird.

5. Durchflußreaktor (1) für Umsetzungen mit Hilfe von Biokatalysatoren, die in Form lyotroper Flüssigkristalle eingesetzt werden, insbesondere für die Herstellung von Cyanhydrinen nach einem der vorangehenden Ansprüche,
**gekennzeichnet durch**
ein Reaktorgehäuse (6), das von einem oder mehreren schmalen Strömungskanälen (2) für substrathaltige Flüssigkeit mit Zu- und Ablaufverteiler (8, 9) eingenommen wird, die zumindest teilweise durch selektiv flüssigkeitsdurchlässige Schichten aus porösem Material gebildet werden und insoweit an flüssigkristallhaltige Schichten (3) angrenzen, deren Abmessungen quer zur Durchflußrichtung eine im wesentlichen vollständige Durchdringung der gesamten flüssigkristallhaltigen Schicht durch reaktives Substrat gestatten.

6. Reaktor nach Anspruch 5,
**gekennzeichnet durch**
einen Stapel von mit Abstand angeordneten länglichen flüssigkeitsdurchlässigen porösen Platten (20), deren Längskanten quer zur Plattenfläche über die Reaktor-Längswände abgeschlossen und miteinander verbunden sind, die durch quer dazu verlaufende Längswände zum Reaktormantel (60) ergänzt werden, dessen zumindest eine Stirnseite Zu- und Ablaufverteiler für substrathaltige Flüssigkeit aufweist, wobei die durch die Abstände gebildeten Lücken durch flüssigkristallhaltige Schichten (30) ausgefüllt werden.

## Claims

1. Process for producing optically active cyanohydrins by enzymatic reaction of oxo compounds with hydrogen cyanide in the presence of (R)- or (S)-oxynitrilase (4.1.2.10) and (4.1.2.11) respectively under such acid conditions that the competing chemical reaction and racemisation are negligible,
characterised in that the reaction is carried out in an organic solvent in the presence of oxynitrilase solubilised in a lyotropic liquid crystal, excluding, in respect of the formation of the liquid crystal, those tensides the hydrolysis of which results in an increased pH.

2. Process according to claim 1,
characterised in that the reaction is carried out in a ternary system comprising tenside/organic solvent/aqueous buffer and prepared using buffer solutions with a pH of between 3 and 6.

3. Process according to claim 1 or 2,
characterised in that the reaction is carried out in a continuous-passage reactor with a liquid crystal fixed on a porous mount, more particularly a glass mount.

4. Process according to any of the preceding claims,
characterised in that the reaction is carried out in a continuous-flow reactor which contains the liquid crystal in a thin laminate adjoining narrow flow ducts incorporating a boundary permeable to liquid and through which solvent or solvent mixture containing substrate is made to flow tangentially to the liquid crystal laminate.

5. Continuous-flow reactor (1) for reactions assisted by biocatalysts which are employed in the form of lyotropic liquid crystals, more particularly for the production of cyanohydrins according to any of the preceding claims,
characterised by a reactor casing (6) which is occupied by one or more narrow flow ducts (2) for liquid containing substrate and having an inflow and an outflow distributor (8, 9) constituted at least in part by layers of porous material selectively permeable to liquids and which in this respect adjoin layers (3) containing liquid crystal and whose dimensions at right angles to the direction of flow permit substantially complete penetration of the entire layer containing liquid crystal by reactive substrate.

6. Reactor according to claim 5,
characterised by a stack of long porous plates (20) permeable to liquid and arranged spaced apart, the long edges of which terminate at right angles to the plate surface via the long walls of the reactor and are joined to one another, which are supplemented by long walls running at right angles thereto to the reactor jacket (60), at least one end face of which incorporates inflow and outflow distributors for liquid containing substrate, the gaps formed by the spaces being filled with layers (30) containing liquid crystal.

## Revendications

1. Procédé de préparation de cyanhydrines optiquement actives, par réaction enzymatique de composés oxo avec l'acide cyanhydrique, en présence de (R)- ou (S)-oxynitrilase (4.1.2.10) ou (4.1.2.11), dans des conditions acides telles que la réaction chimique de concurrence et la racémisation sont négligeables, caractérisé en ce qu'on effectue la réaction dans un solvant organique, en présence d'une oxynitrilase dissoute dans un cristal liquide lyotrope, et dans lequel, pour la formation du cristal liquide, on exclut les agents tensio-actifs dont l'hydrolyse conduit à une augmentation du pH.

2. Procédé selon la revendication 1, caractérisé en ce qu'on effectue la réaction dans un système ternaire d'agent tensio-actif/solvant organique/tampon aqueux, dont la valeur de pH a été ajustée entre 3 et 6 grâce à l'utilisation de solutions tamponnées.

3. Procédé selon la revendication 1 ou 2, caractérisé en ce qu'on effectue la réaction dans un réacteur à régime continu avec des cristaux liquides fixés sur un support poreux, en particulier un support en verre.

4. Procédé selon l'une des revendications précédentes, caractérisé en ce qu'on effectue la réaction dans un réacteur à régime continu qui contient les cristaux liquides dans une couche mince contiguë à des canaux étroits d'écoulement, présentant une limitation de la perméabilité aux liquides, par lesquels le solvant ou le mélange de solvants contenant le substrat est mis par écoulement, de manière tangentielle par rapport aux couches de cristaux liquides.

5. Réacteur à régime continu (1) pour des réactions à l'aide de biocatalyseurs qui sont utilisés sous forme de cristaux liquides lyotropes, en particulier pour la préparation de cyanhydrines selon l'une des revendications précédentes, caractérisé en ce qu'il comporte une enceinte de réacteur (6) comprenant un ou plusieurs canaux d'écoulement (2) étroits pour le liquide contenant le substrat, avec un distributeur d'amenée et un distributeur d'écoulement (8, 9), qui sont formés, au moins en partie, par des couches de matériau poreux laissant passer le liquide de façon sélective et sont contigus à des couches (3) contenant les cristaux liquides, dont les dimensions perpendiculairement à la direction d'écoulement permettent à l'ensemble de la couche contenant les cristaux liquides d'être pénétrée de manière essentiellement complète par le substrat apte à réagir.

6. Réacteur selon la revendication 5, caractérisé en ce qu'il comporte une pile de plaques poreuses (20) allongées, laissant passer les liquides et disposées à distance les unes des autres, dont les grands côtés sont fermés par l'intermédiaire des parois longitudinales du réacteur, par rapport à la face des plaques, et reliés entre eux, qui sont complétés par des parois longitudinales dans la direction de l'enveloppe (60) du réacteur, orientées perpendiculairement, dont au moins un côté frontal présente des distributeurs d'amenée et d'évacuation pour le liquide contenant le substrat, les vides formés par les intervalles étant comblés par des couches (30) contenant les cristaux liquides.
